# EUROPEAN PATENT SPECIFICATION

(11) **EP 0 468 562 B1**
(45) Date of publication and mention of the grant of the patent: **08.12.1993**
(21) Application number: 91201688.8
(22) Date of filing: 02.07.1991
(51) Int. Cl.: C07D 417/04, C07D 401/04, C07D 413/04, C07D 495/04, C07D 498/04, C07D 513/04, A61K 31/55

(54) **Tetrahydropyridinyl substituted tricyclic derivatives with dopamine antagonistic activity**
Tetrahydropyridinyl substituierte tricyclische Derivate mit Dopamin antagonistischer Wirkung
Dérivés tricycliques substitués par un reste tétrahydropyridinyle à activité antagoniste de la dopamine

(30) Priority: 24.07.1990 EP 90308071
(43) Date of publication of application: 29.01.1992
(73) Proprietor: Akzo Nobel N.V., 6824 BM Arnhem (NL)
(72) Inventor: Cairns, James, Cumbernauld G67, Lanarkshire Scotland (GB); Gibson, Samuel George, Motherwell ML1 3AU, Lanarkshire Scotland (GB); Rae, Duncan Robertson, Lanark ML11 7GR, Lanarkshire Scotland (GB)
(74) Representative: Hermans, Franciscus G.M.

(56) References cited:
- EP-A- 0 291 673
- US-A- 3 501 483
- US-A- 4 221 714

## Description

The invention relates to tetrahydropyridinyldibenzazepine derivatives of formula I 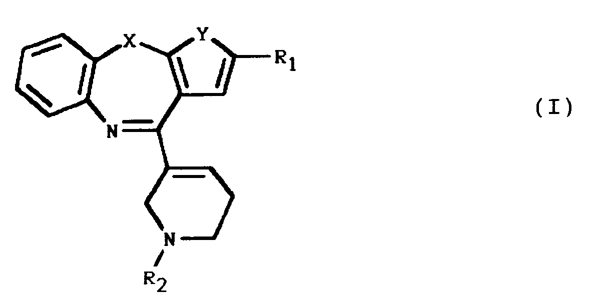
wherein
R₁ is a substituent selected from the group consisting of hydrogen, halogen, and alkyl having 1-4 carbon atoms;
R₂ is selected from hydrogen, unsubstituted or hydroxy substituted alkyl having 1-4 carbon atoms, and unsubstituted or hydroxy substituted alkenyl having 2-6 carbon atoms;
X is O, S, or CH₂;
Y is HC=CH or S;
or a pharmaceutically acceptable salt thereof.

These compounds are dopamine antagonists, and are therefore useful for the treatment of psychotic disorders such as schizophrenia, schizophreniform disorder, delusional disorder, acute manic states, senile and presenile psychotic states, toxic psychoses, psychoactive-substance induced psychoses, symptoms of severe restlessness, hyperexcitability and anxiety, motor disorders, among which Tourette's disorder and Huntington's chorea, nausea and vomiting, hiccup and dizziness.

Related compounds are known from USP 4,221,714, which differ from the present compounds in having a 4-tetrahydropyridinyl group, instead of a 3-tetrahydropyridinyl group. All the compounds disclosed in said patent are, moreover, 8-chloro derivatives, whereas the corresponding aromatic ring in the present compounds is unsubstituted. These prior art compounds are claimed as antipsychotic agents, but are known to have cardiovascular and extra-pyramidal side-effects. The compounds of formula I have lower affinity to α₁-adrenergic receptors, and a more favourable binding to dopamine D₂ receptors than to α₁-adrenergic receptors (spiperone-prazosin binding ratio) than the corresponding prior art compounds, indicating that the compounds of the invention exhibit less cardiovascular side-effects, especially less orthostatic hypotension. Most of the compounds of this invention, moreover, show significant improvements by having stronger binding to muscarinic-cholinergic than to dopamine D₂ receptors (as demonstrated by the 3-quinuclidinyl benzilate and spiperone binding affinities), which results in less extra-pyramidal side-effect. The compounds of this invention, thus, have fewer cardiovascular, and mostly also fewer extra-pyramidal side-effects. Other related compounds are disclosed in USP 3,501,483, but these compounds do not display antipsychotic activity.

The term halogen used in the definition of formula I means fluorine, chlorine, bromine or iodine. Chlorine is the preferred halogen.

The term alkyl means a branched or unbranched alkyl group with 1-4 carbon atoms, such as methyl, ethyl, propyl, isopropyl, butyl, *tert*-butyl and the like. Preferred alkyl groups are methyl and ethyl.

The term alkenyl means a branched or unbranched aliphatic hydrocarbon group with 2-6 carbon atoms having a double bond. Examples are ethenyl, 1-propenyl, 2-propenyl, 2-butenyl, 2-methyl-2-propenyl and 2,3-dimethyl-1,3-butadienyl.

Preferred compounds according to the invention are compounds having formula I, in which R₁ is chlorine, bromine or ethyl, and R₂ is methyl or ethyl, or a pharmaceutically acceptable salt thereof.

Preferably, Y is HC=CH and X is S, or Y is S and X is CH₂.

The most preferred compound has formula I, in which R₁ is chlorine, R₂ is methyl, X is S, and Y is HC=CH, or a pharmaceutically acceptable salt thereof.

The compounds of the invention can be prepared by methods commonly in use for the preparation of similar known compounds. A suitable method of preparation is the cyclodehydration of an amide of formula II 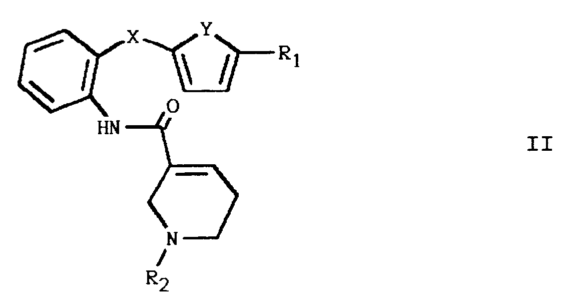
in which R₁, R₂, X and Y have the previously given meanings.
This reaction, which is generally known as the Bischler-Napieralski reaction, can be performed by means of condensation agents such as phosphorous pentoxide, zinc chloride, polyphosphoric acid, phosphoryl chloride, or mixtures thereof.

Alternatively an imidoyl halide of the formula III 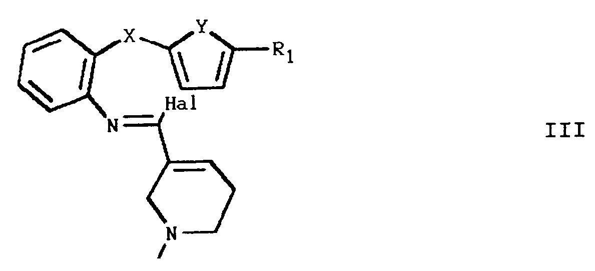
in which R₁, R₂, X and Y have the previously given meanings and Hal denotes a halide, and preferably chlorine, is cyclized by means of Lewis acids such as phosphorous pentachloride, zinc chloride, aluminum trichloride, boron trifluoride, or mixtures thereof.

Another method of preparation of compounds of formula I is the reduction of pyridinium derivatives of formula IV 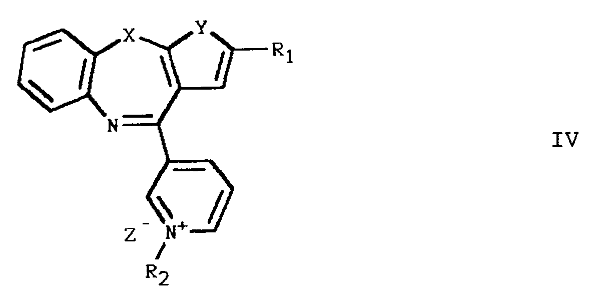
in which R₁, R₂, X and Y have the previously given meanings and Z is a leaving group, such as a halide (preferably chlorine), or sulphonate. A suitable reduction means is for instance sodium borohydride.
Compounds of formula IV can be prepared by reaction of the corresponding pyridinyl derivative V 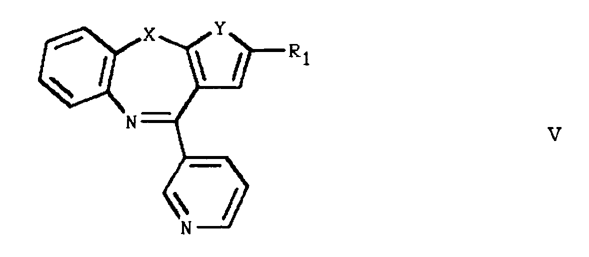
with R₂-Z, in which R₁, R₂, X, Y, and Z have the previously given meanings.

The novel compounds of formula I may be isolated from a reaction mixture in the form of a pharmaceutically acceptable salt. The pharmaceutically acceptable salts may also be obtained by treating the free base of formula I with an organic or inorganic acid such as HCl, HBr, HI, H₂SO₄, H₃PO₄, acetic acid, propionic acid, glycolic acid, maleic acid, malonic acid, methanesulphonic acid, fumaric acid, succinic acid, tartaric acid, citric acid, benzoic acid, and ascorbic acid.

The compounds of the invention may be administered enterally or parenterally, and for humans preferably in a daily dosage of 0,001-10 mg per kg body weight. Mixed with pharmaceutically suitable auxiliaries, e.g. as described in the standard reference, Chase et al., Remington's Pharmaceutical Sciences, the compounds may be compressed into solid dosage units, such as pills, tablets, or be processed into capsules or suppositories. By means of pharmaceutically suitable liquids the compounds can also be applied as an injection preparation in the form of a solution, suspension, emulsion, or as a spray, e.g. a nasal spray.

The invention is further illustrated by the following examples.
The numbering of the compounds is as follows:

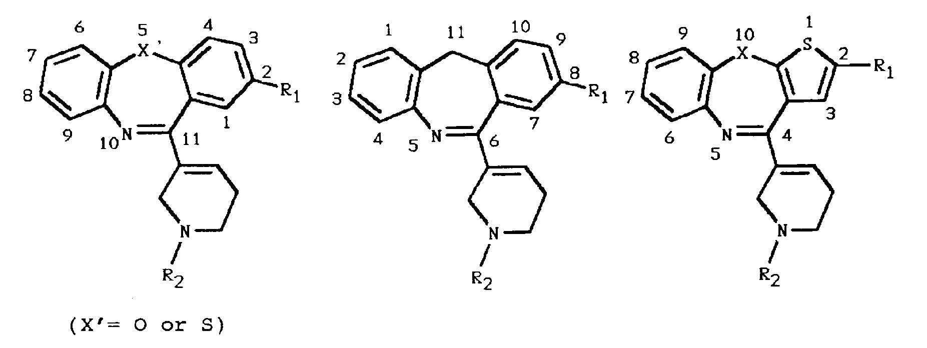

### Example 1

### 2-chloro-11-(1,2,5,6-tetrahydro-1-methyl-3-pyridinyl)dibenzo[b,f][1,4]thiazepine (E)-2-butenedioate (1:1)

A mixture of 3,7 g of N-[2-(4-chlorophenylthio)phenyl]-1-methyl-1,2,5,6-tetrahydro-3-pyridinecarboxamide, 37 g of polyphosphoric acid and 3,7 ml of phosphorus oxychloride was heated at 110 °C for 45 minutes. Water was added and the solution was basified with potassium hydroxide. The product was extracted with dichloromethane to give a gum which was crystallized from methanol to give 1,05 g of a product that was converted to the fumarate salt, which on crystallization from methanol gave 2-chloro-11-(1,2,5,6-tetrahydro-1-methyl-3-pyridinyl)dibenzo[b,f][1,4]thiazepine (E)-2-butenedioate (1:1), m.p. 199 °C (dec.).

### Example 2

In an analogous manner as described in Example 1 were prepared:

8-chloro-6-(1,2,5,6-tetrahydro-1-methyl-3-pyridinyl)-11H-dibenz[b,e]azepine (E)-2-butenedioate (1:1), m.p. 198-201 °C (dec.).

2-chloro-11-(1,2,5,6-tetrahydro-3-pyridinyl)dibenzo[b,f][1,4]thiazepine, m.p. 88-90 °C.

2-chloro-11-(1,2,5,6-tetrahydro-1-methyl-3-pyridinyl)dibenz[b,f][1,4]oxazepine (E)-2-butenedioate (2:1), m.p. 175.3 °C.

2-bromo-11-(1,2,5,6-tetrahydro-1-methyl-3-pyridinyl)dibenzo[b,f][1,4]thiazepine 2,3-dihydroxybutanedioate (2:3), m.p. 124 °C (dec.).

2-bromo-11-(1,2,5,6-tetrahydro-1-methyl-3-pyridinyl)dibenz[b,f][1,4]oxazepine 2,3-dihydroxybutanedioate (2:3), m.p. 160.1 °C (dec.).

2-chloro-11-(1-ethyl-1,2,5,6-tetrahydro-3-pyridinyl)dibenzo[b,f][1,4]thiazepine (E)-2-butenedioate (1:1), m.p. 195-198 °C.

2-ethyl-11-(1,2,5,6-tetrahydro-1-methyl-3-pyridinyl)dibenz[b,f][1,4]oxazepine (E)-2-butenedioate (1:1), m.p. 125.7 °C (dec.).

2-ethyl-11-(1,2,5,6-tetrahydro-1-methyl-3-pyridinyl)dibenzo[b,f][1,4]thiazepine (E)-2-butenedioate (1:1), m.p. 125.1 °C (dec.).

6-(1,2,5,6-tetrahydro-1-methyl-3-pyridinyl)-11H-dibenz[b,e]azepine (Z)-2-butenedioate (1:1), m.p. 202.8 °C.

### Example 3

In a similar manner as described in Example 1, but using PPE (polyphosphoric ester) instead of polyphosphoric acid and phosphorus oxychloride were prepared:

2-chloro-4-(1,2,5,6-tetrahydro-1-methyl-3-pyridinyl)-10H-thieno[3,2-c]benzazepine ethanedioate (1:1), m.p. 202.4 °C.

2-methyl-4-(1,2,5,6-tetrahydro-1-methyl-3-pyridinyl)-10H-thieno[3,2-c]benzazepine (E)-2-butenedioate (1:1), m.p. 198.1 °C.

2-ethyl-4-(1,2,5,6-tetrahydro-1-methyl-3-pyridinyl)thieno[3,2-f]benzo[1,4]thiazepine ethanedioate (1:1).

## Claims (Claims for the following Contracting State(s): AT, BE, CH, DE, DK, FR, GB, IT, LI, NL, SE)

1. A tetrahydropyridinyldibenzazepine derivatives of formula I 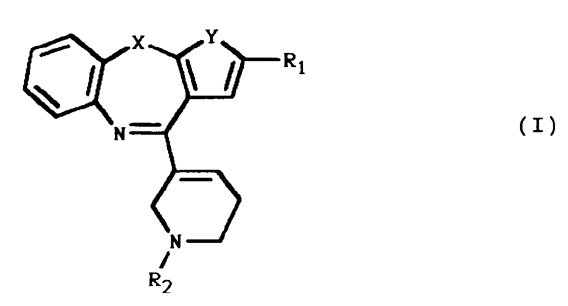 wherein
R₁ is a substituent selected from the group consisting of hydrogen, halogen, and alkyl having 1-4 carbon atoms;
R₂ is selected from hydrogen, unsubstituted or hydroxy substituted alkyl having 1-4 carbon atoms, and unsubstituted or hydroxy substituted alkenyl having 2-6 carbon atoms;
X is O, S, or CH₂;
Y is HC=CH or S;
or a pharmaceutically acceptable salt thereof.

2. The tetrahydropyridinyldibenzazepine derivative of claim 1, characterized in that R₁ is chlorine, bromine or ethyl, and R₂ is methyl or ethyl, or a pharmaceutically acceptable salt thereof.

3. The tetrahydropyridinyldibenzazepine derivative of claim 1 or 2, characterized in that Y is HC=CH and X is S, or Y is S and X is CH₂, or a pharmaceutically acceptable salt thereof.

4. The tetrahydropyridinyldibenzazepine derivative of claim 1, characterized in that R₁ is chlorine, R₂ is methyl, X is S, and Y is HC=CH, or a pharmaceutically acceptable salt thereof.

5. A process for the preparation of the tetrahydropyridinyldibenzazepine of claim 1 , characterized in that
a) an amide of formula II 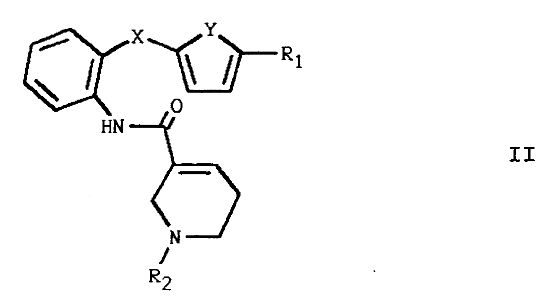 wherein R₁, R₂, X, and Y have the previously given meanings is cyclized, or
b) an imidoyl halide of formula III 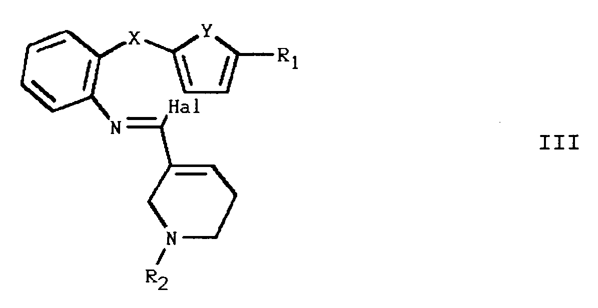 in which R₁, R₂, X, and Y have the previously given meanings and Hal denotes a halide, is cyclized;
or
c) a pyridinium derivatives of formula IV 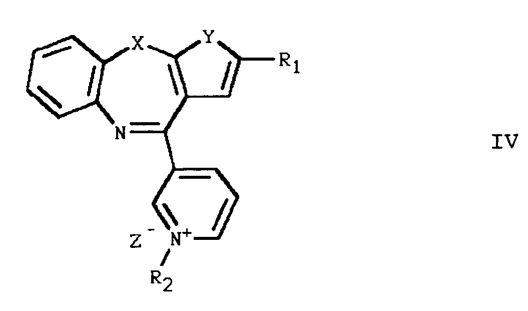 in which R₁, R₂, X, and Y have the previously given meanings and Z is a leaving group, is reduced;
after which the product obtained is optionally converted into a pharmaceutically acceptable salt.

6. A pharmaceutical preparation comprising the tetrahydropyridinyldibenzazepine derivative of claim 1 in admixture with pharmaceutically acceptable auxiliaries.

7. A use of the tetrahydropyridinyldibenzazepine derivative of claim 1 for the manufacture of a medicament for the treatment of psychotic disorders.

8. The tetrahydropyridinyldibenzazepine derivative of any one of claims 1-4 for use in therapy.

## Claims (Claims for the following Contracting State(s): ES, GR)

1. A process for the preparation of a tetrahydropyridinyldibenzazepine derivative of formula I 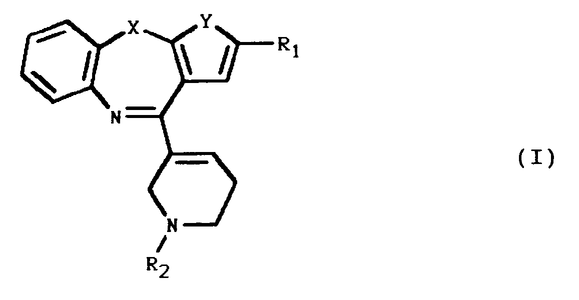 wherein
R₁ is a substituent selected from the group consisting of hydrogen, halogen, and alkyl having 1-4 carbon atoms;
R₂ is selected from hydrogen, unsubstituted or hydroxy substituted alkyl having 1-4 carbon atoms, and unsubstituted or hydroxy substituted alkenyl having 2-6 carbon atoms;
X is O, S, or CH₂;
Y is HC=CH or S;
or a pharmaceutically acceptable salt thereof,
characterized in that
a) an amide of formula II 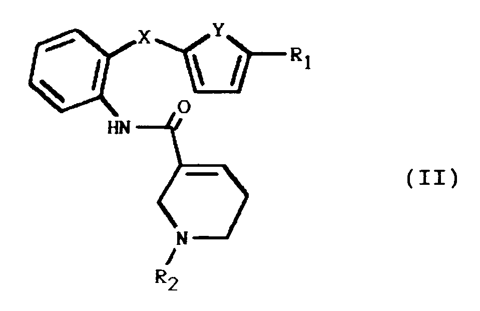 wherein R₁, R₂, X, and Y have the previously given meanings is cyclized, or
b) an imidoyl halide of formula III 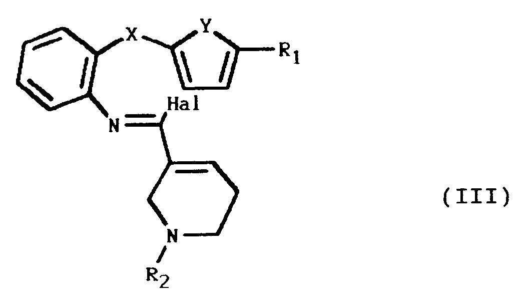 in which R₁, R₂, X, and Y have the previously given meanings and Hal denotes a halide, is cyclized, or
c) a pyridinium derivative of formula IV 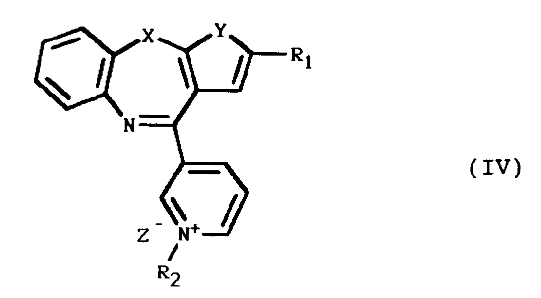 in which R₁, R₂, X and Y have the previously given meanings and Z is a leaving group, is reduced; after which the product obtained is optionally converted into a pharmaceutically acceptable salt.

2. The process according to claim 1, wherein R₁ is chlorine, bromine or ethyl, and R₂ is methyl or ethyl.

3. The process according to claim 1 or 2, wherein Y is HC=CH and X is S, or Y is S and X is CH₂.

4. The process according to claim 1, wherein R₁ is chlorine, R₂ is methyl, X is S, and Y is HC=CH.

## Patentansprüche (Patentansprüche für folgende(n) Vertragsstaat(en): AT, BE, CH, DE, DK, FR, GB, IT, LI, NL, SE)

1. Tetrahydropyridinyldibenzazepin-derivate der Formel I 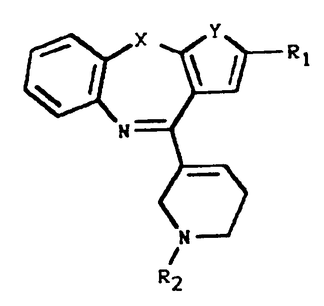 in welcher
R₁ ein Substituent, ausgewählt aus der Gruppe bestehend aus Wasserstoff, Halogen und Alkyl mit 1-4 Kohlenstoffatomen, ist;
R₂ ausgewählt ist aus Wasserstoff, unsubstituiertem oder hydroxy-substituiertem Alkyl mit 1-4 Kohlenstoffatomen und unsubstituiertem oder hydroxy-substituiertem Alkenyl mit 2-6 Kohlenstoffatomen;
X O, S oder CH₂ ist;
Y HC=CH oder S ist;
oder ein pharmazeutisch annehmbares Salz davon.

2. Das Tetrahydropyridinyldibenzazepin-derivat nach Patentanspruch 1, dadurch gekennzeichnet, dass R₁ Chlor, Brom oder Aethyl ist und R₂ Methyl oder Aethyl ist, oder ein pharmazeutisch annehmbares Salz davon.

3. Das Tetrahydropyridinyldibenzazepin-derivat nach Patentanspruch 1 oder 2, dadurch gekennzeichnet, dass Y HC=CH ist und X S ist oder Y S ist und X CH₂ ist, oder ein pharmazeutisch annehmbares Salz davon.

4. Das Tetrahydropyridinyldibenzazepin-derivat nach Patentanspruch 1, dadurch gekennzeichnet, dass R₁ Chlor ist, R₂ Methyl ist, X S ist und Y HC=CH ist, oder ein pharmazeutisch annehmbares Salz davon.

5. Ein Verfahren zur Herstellung des Tetrahydropyridinyldibenzazepins nach Patentanspruch 1, dadurch gekennzeichnet, dass
a) ein Amid der Formel II 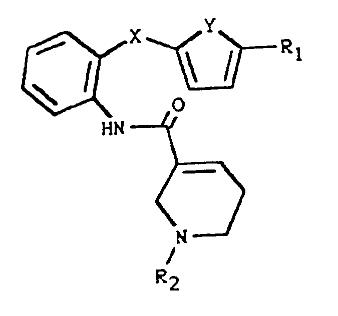 in welchem R₁, R₂, X und Y die oben gegebenen Bedeutungen aufweisen, zyklisiert wird, oder
b) ein Imidoylhalogenid der Formel III 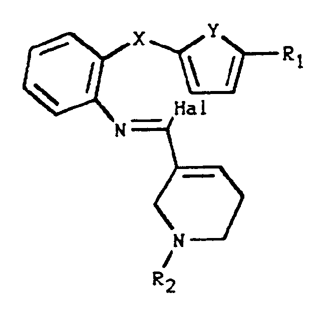 in welcher R₁, R₂, X und Y die oben gegebenen Bedeutungen aufweisen und Hal ein Halogenid bedeutet, zyklisiert wird; oder
c) ein Pyridiniumderivat der Formel IV 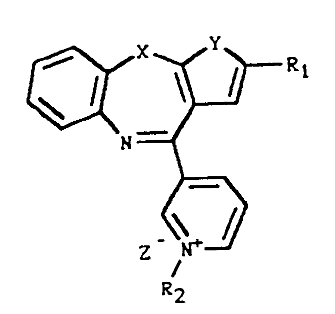 in welcher R₁, R₂, X und Y die oben gegebenen Bedeutungen aufweisen und Z eine ausscheidende Gruppe ist, reduziert wird;
worauf das erhaltene Produkt gegebenenfalls in ein pharmazeutisch annehmbares Salz umgewandelt wird.

6. Ein pharmazeutisches Präparat, welches das Tetrahydropyridinyldibenzazepin-derivat nach Patentanspruch 1 im Gemisch mit pharmazeutisch annehmbaren Hilfsstoffen enthält.

7. Eine Verwendung des Tetrahydropyridinyldibenzazepin-derivats nach Patentanspruch 1 zur Herstellung eines Medikamentes für die Behandlung psychotischer Erkrankungen.

8. Das Tetrahydropyridinyldibenzazepin-derivat nach einem der Patentansprüche 1 bis 4 zur Verwendung in der Therapie.

## Patentansprüche (Patentansprüche für folgende(n) Vertragsstaat(en): ES, GR)

1. Ein Verfahren zur Herstellung eines Tetrahydropyridinyldibenzazepin-derivates der Formel I 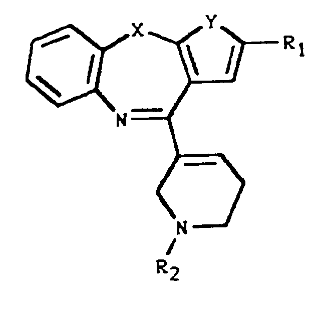 in welcher
R₁ ein Substituent ausgewählt aus der Gruppe bestehend aus Wasserstoff, Halogen und Alkyl mit 1-4 Kohlenstoffatomen ist;
R₂ ausgewählt ist aus Wasserstoff, unsubstituiertem oder hydroxy-substituiertem Alkyl mit 1-4 Kohlenstoffatomen und unsubstituiertem oder hydroxy-substituiertem Alkenyl mit 2-6 Kohlenstoffatomen;
X O, S oder CH₂ ist;
Y HC=CH oder S ist;
oder eines pharamzeutisch annehmbaren Salzes davon,
dadurch gekennzeichnet, dass
a) ein Amid der Formel II 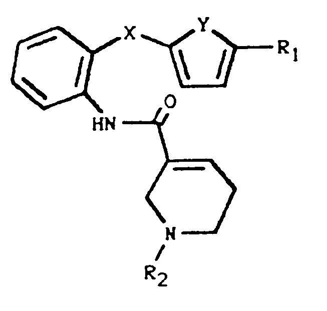 in welchem R₁, R₂, X und Y die oben gegebenen Bedeutungen aufweisen, zyklisiert wird, oder
b) ein Imidoylhalogenid der Formel III 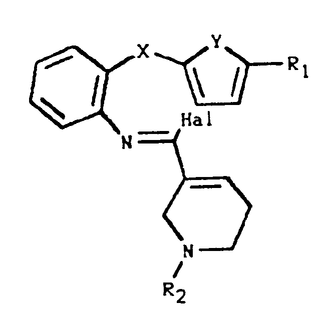 in welcher R₁, R₂, X und Y die oben gegebenen Bedeutungen aufweisen und Hal ein Halogenid bedeutet, zyklisiert wird; oder
c) ein Pyridinium-derivat der Formel IV 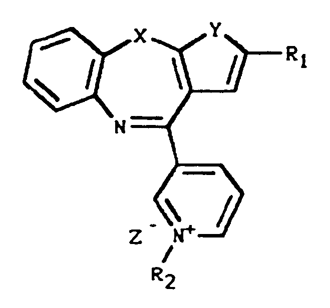 in welcher R₁, R₂, X und Y die oben gegebenen Bedeutungen aufweisen und Z eine ausscheidende Gruppe ist, reduziert wird;
worauf das erhaltene Produkt gegebenenfalls in ein pharmazeutisch annehmbares Salz umgewandelt wird.

2. Das Verfahren nach Patentanspruch 1, in welchem R₁ Chlor, Brom oder Aethyl ist und R₂ Methyl oder Aethyl ist.

3. Das Verfahren nach Patentanspruch 1 oder 2, in welchem Y HC=CH und X S ist oder Y S ist und X CH₂ ist.

4. Das Verfahren nach Patentanspruch 1, in welchem R₁ Chlor ist, R₂ Methyl ist, X S ist und Y HC=CH ist.

## Revendications (Revendications pour l'(les) Etat(s) contractant(s) suivant(s): AT, BE, CH, DE, DK, FR, GB, IT, LI, NL, SE)

1. Un dérivé de tétrahydropyridinyl-dibenzazépine de formule I: 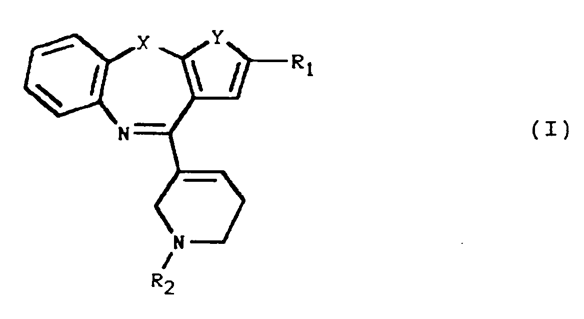 dans laquelle:
R₁ est un substituant choisi dans le groupe constitué par un atome d'hydrogène, d'halogène, et un radical alkyle renfermant de 1 à 4 atomes de carbone;
R₂ est choisi parmi un atome d'hydrogène, un radical alkyle non substitué ou substitué par un groupe hydroxy et renfermant de 1 à 4 atomes de carbone, et un groupe alkényle non substitué ou substitué par un groupe hydroxy et renfermant de 2 à 6 atomes de carbone;
X représente O, S ou CH₂;
Y représente HC=CH ou S;
ou un de ses sels pharmaceutiquement acceptable.

2. Le dérivé de tétrahydropyridinyldibenzazépine selon la revendication 1, caractérisé en ce que:
R₁ représente un atome de chlore, de brome ou un radical éthyle, et
R₂ représente méthyle ou éthyle,
ou un de ses sels pharmaceutiquement acceptable.

3. Le dérivé de tétrahydropyridinyldibenzazépine selon la revendication 1 ou 2, caractérisé en ce que:
Y représente HC=CH et X représente S,
ou
Y représente S et X représente CH₂,
ou un de ses sels pharmaceutiquement acceptable.

4. Le dérivé de tétrahydropyridinyldibenzazépine selon la revendication 1, caractérisé en ce que:
R₁ représente un atome de chlore,
R₂ représente le radical méthyle,
X représente S et
Y représente HC=CH,
ou un de ses sels pharmaceutiquement acceptable.

5. Un procédé de préparation de tétrahydropyridinyldibenzazépine selon la revendication 1, caractérisé en ce que:
a) un amide de formule II: 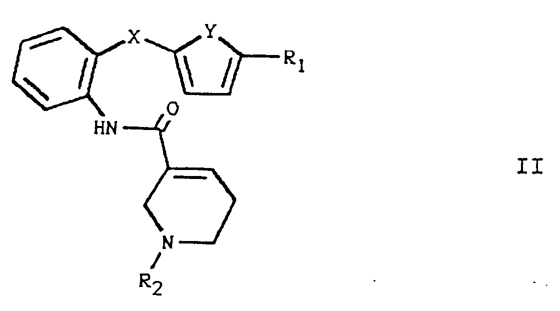 dans laquelle R₁, R₂, X et Y ont les significations indiqués ci-dessus est cyclisé, ou
b) un halogénure d'imidoyle de formule III: 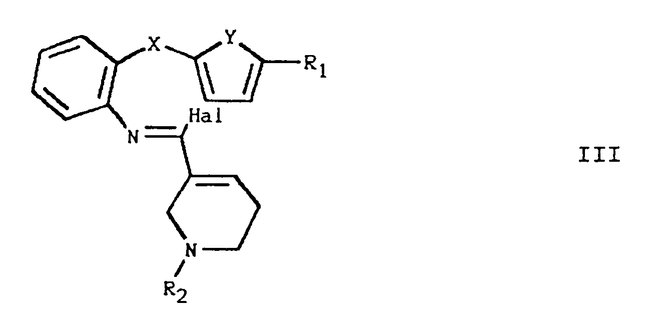 dans laquelle R₁, R₂, X et Y ont les significations indiqués ci-dessus et Hal dénote un halogénure, est cyclisé;
ou
c) un dérivé de pyridinium de formule IV: 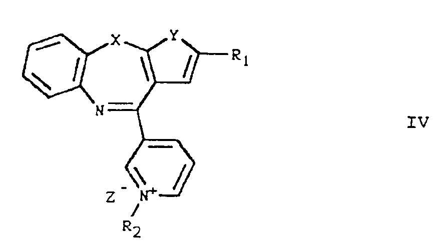 dans laquelle R₁, R₂, X et Y ont les significations indiqués ci-dessus, et Z représente un groupe partant, est réduit;
ce après quoi le produit obtenu est éventuellement converti en un sel pharmaceutiquement acceptable.

6. Préparation pharmaceutique comprenant le dérivé de tétrahydropyridinyldibenzazépine selon la revendication 1, en mélange avec des auxiliaires pharmaceutiquement acceptables.

7. Une utilisation d'un dérivé de tétrahydropyridinyl-dibenzazépine selon la revendication 1, pour la préparation d'un médicament destiné au traitement de désordres psychotiques.

8. Le dérivé de tétrahydropyridinyldibenzazépine selon l'une quelconque des revendications 1 à 4, pour son utilisation thérapeutique.

## Revendications (Revendications pour l'(les) Etat(s) contractant(s) suivant(s): ES, GR)

1. Un procédé de préparation d'un dérive de tétrahydropyridinyl-dibenzazépine de formule I: 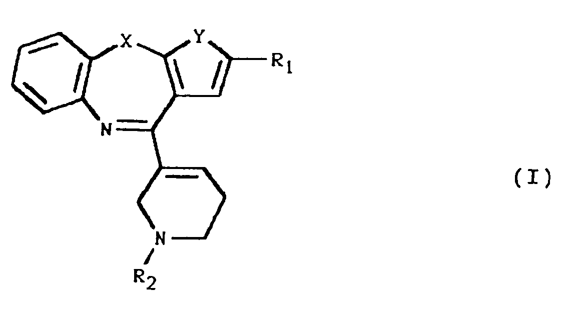 dans laquelle:
R₁ est un substituant choisi dans le groupe constitué par un atome d'hydrogène, d'halogène, et un radical alkyle renfermant de 1 à 4 atomes de carbone;
R₂ est choisi parmi un atome d'hydrogène, un radical alkyle non substitué ou substitué par un groupe hydroxy et renfermant de 1 à 4 atomes de carbone, et un groupe alkényle non substitué ou substitué par un groupe hydroxy et renfermant de 2 à 6 atomes de carbone;
X représente O, S ou CH₂;
Y représente HC=CH ou S;
ou un de leurs sels pharmaceutiquement acceptable,
caractérisé en ce que:
a) un amide de formule II: 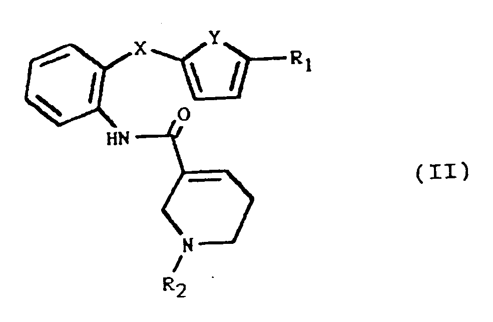 dans laquelle R₁, R₂, X et Y ont les significations indiqués ci-dessus est cyclisé, ou
b) un halogénure d'imidoyle de formule III: 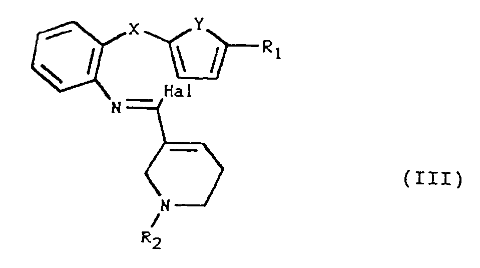 dans laquelle R₁, R₂, X et Y ont les significations indiqués ci-dessus et Hal dénote un halogénure, est cyclisé;
ou
c) un dérivé de pyridinium de formule IV: 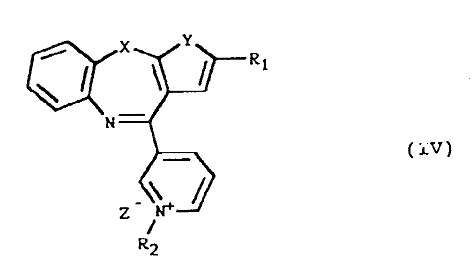 dans laquelle R₁, R₂, X et Y ont les significations indiqués ci-dessus, et Z est un groupe partant, est réduit;
ce après quoi le produit obtenu est éventuellement converti en un sel pharmaceutiquement acceptable.

2. Le procédé selon la revendication 1, dans lequel R₁ représente un atome de chlore, de brome ou un radical éthyle, et R₂ est un radical méthyle ou éthyle.

3. Le procédé selon la revendication 1 ou 2, dans lequel Y est HC=CH et X représente S, ou Y représente S et X représente CH₂.

4. Le procédé selon la revendication 1, dans lequel R₁ est un atome de chlore, R₂ est un radical méthyle, X représente S et Y représente HC=CH.
